# EUROPEAN PATENT SPECIFICATION

(11) **EP 2 912 509 B1**
(45) Date of publication and mention of the grant of the patent: **12.04.2017**
(21) Application number: 13779602.5
(22) Date of filing: 22.10.2013
(51) Int. Cl.: G02B 19/00, F21V 8/00, A61N 5/06, F21V 5/00

(54) **DEVICE, APPARATUS AND METHOD FOR COSMETIC TREATMENT BY LIGHT**
VORRICHTUNG, EINRICHTUNG UND VERFAHREN ZUR KOSMETISCHEN BEHANDLUNG DURCH LICHT
DISPOSITIF, APPAREIL ET MÉTHODE DE TRAITEMENT COSMÉTIQUE PAR LA LUMIÈRE

(30) Priority: 23.10.2012 FR 1260086; 24.01.2013 US 201361756047 P
(43) Date of publication of application: 02.09.2015
(73) Proprietor: L'Oréal, 75008 Paris (FR)
(72) Inventor: GUGLIELMI, Valérie, F-75008 Paris (FR); PLANARD-LUONG, Thi Hong Lien, F-91440 Bures sur Yvette (FR)
(74) Representative: Jouanneau, Lionel
(86) International application number: PCT/EP2013/072024
(87) International publication number: WO 2014/064075

(56) References cited:
- US-A1- 2002 191 396
- US-A1- 2005 049 582
- US-A1- 2005 083 687
- US-A1- 2006 071 245
- US-A1- 2006 293 727
- US-A1- 2008 212 319
- US-A1- 2010 027 257
- US-A1- 2010 039 812
- US-A1- 2010 207 131
- US-A1- 2010 331 929

## Description

### Context of the invention

The present invention relates to a device for cosmetic treatment of the skin, i.e. non-therapeutic, especially by exposing an area of skin to light. The present invention also relates to an apparatus comprising such a device and a cosmetic method comprising the use of such an apparatus.

### Discussion of the prior art

There exist apparatuses for dermatological treatment of the skin by luminotherapy. For example, document US-A-7 887 533 describes such an apparatus for professional use, by doctors especially. This apparatus comprises a system for assembling light-emitting diodes (LEDs) arranged in rows and coupled with cylindrical lenses. The lenses make it possible to tailor the angle of radiation of each light beam of the LEDs with greater or lesser flexibility. Document WO-A-2012/086991 describes an apparatus using several wavelengths concomitantly to treat various pathologies. Further light treatment systems employing LED arrays cooperating with optical screens are disclosed in US2008/0212319 A1 and US2010/0039812 A1. There also exist apparatuses for cosmetic treatment of the skin by exposure to light which are for private use. A user holds the apparatus in their hand and orients it so as to expose a given area of skin to a luminous radiation emitted by LEDs arranged in the apparatus. It is for example possible to cite the devices described in documents FR-A-2 917 299, US-A-2010/274329 or WO-A-2008/057640. The known apparatuses typically comprise one or more LEDs disposed in a casing. The number of LEDs is often significant so as to guarantee homogeneity of the treatment and the emission wavelength of the LEDs is chosen according to the applications envisaged. For example, document EP-A-1 648 385 proposes to use at least one first source emitting yellow light (with a dominant wavelength at about 590 nm) and at least one second source emitting infrared light (with a dominant wavelength at about 850 nm) with controlled powers for each source.

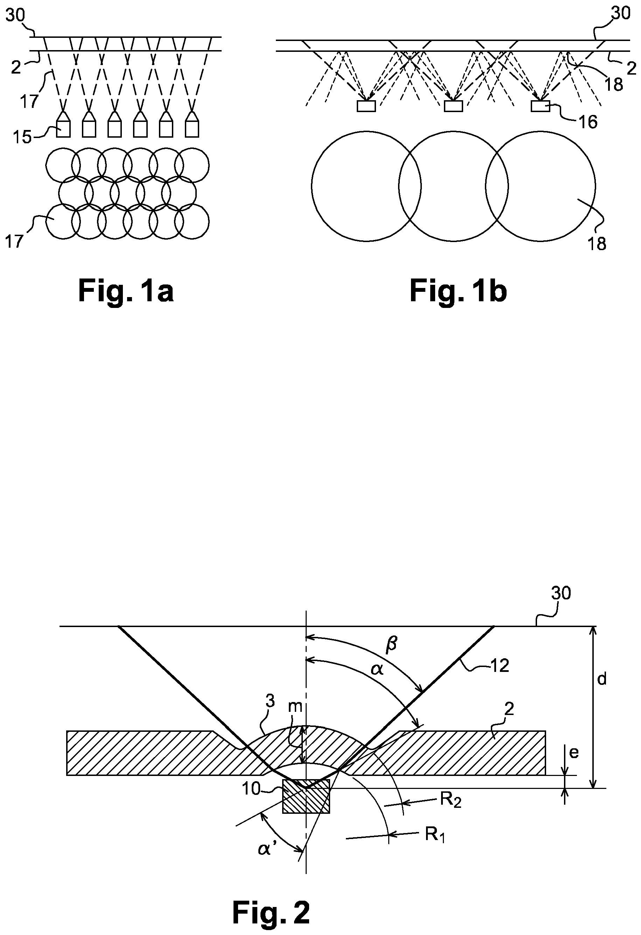

Figures 1a and 1b schematically illustrate the radiation distributions obtained on an area of skin 30 exposed to light with various types of light-emitting diodes 15, 16. For example, so-called "conventional" LEDs 15 (Figure 1a) emit a light beam 17 with a relatively small angular aperture, of the order of 15° to 20°, and with a homogeneous power of the radiation over the exposed area 30. There must nonetheless be multiple such LEDs 15 in order to homogeneously cover the whole of an area of skin to be treated. On the other hand, so-called "power" LEDs 16 (Figure 1b) emit a light beam 18 with a relatively large angular aperture, of the order of 120° to 140° - i.e. 60° to 70° in terms of angle of impact.

By comparison with conventional LEDs, power LEDs are efficacious in terms of efficiency, lifetime and compactness. The use of such HPLEDs (High-power LEDs or HPLEDs) raises certain problems however. Firstly, having regard to their use, it is necessary to protect the LEDs by a screen 2 transparent to the light beams 17, 18 emitted. Under these conditions, the HPLEDs 16 lose part of their radiation power, in particular on the peripheral area, because of the high angle of impact which generates significant reflection on the internal surface of the screen 2, as illustrated on the right of Figure 1. Moreover, the use of several LEDs in a concomitant manner poses a problem of homogeneity of the radiations. Indeed, the light beam 17, 18 emitted by an LED exhibits a pointlike radiation power over the whole of the illuminated area 30 that decreases with the angle of impact of each light ray of the beam. In the case of conventional LEDs, as the angle of impact is small, the radiation power is substantially homogeneous over the whole of the illuminated area; but in the case of HPLEDs, on account of their large angular aperture, the radiation power is not homogeneous over the whole of the illuminated area.

These problems can be solved in all or part with technologies of diffusers such as "backlights" for the screens, as described with reference to Figure 5 of the document KR-A-10-1085937, and/or with diffusion lenses. Nonetheless, such solutions are expensive.

### Summary of the invention

There therefore exists a need for a device for cosmetic treatment by light which makes it possible to optimize the efficiency of the LEDs while ensuring homogeneity of the radiations and which is inexpensive.

For this purpose, the invention proposes to integrate into the screen positioned opposite the LEDs, imprints acting as lenses so as to modify the angular aperture of the emitted beams. These imprints are formed (for example etched) in the thickness of the screen itself. Such a solution is cheap and makes it possible to ensure that the light beam emitted by each LED is transmitted to the area to be treated with homogeneous power. It is possible to envisage an imprint per LED or an imprint for certain LEDs only. Moreover, the screen comprising said imprints constitutes the exterior interface of the device and makes it possible to protect the LEDs from the exterior environment.

In the case of a combination of various types of LED emitting radiations at various wavelengths and with different required powers, it would be possible to use the same number of LEDs of each type distributed homogeneously over a given surface so as to ensure homogeneous exposure of the area to be treated to both types of radiations. Such a solution is expensive since it exhibits significant losses of efficiency for the group of LEDs having to emit with a reduced power. Another solution would be to use expensive multiple components (synchronous or sequential RGB).

The invention makes it possible to alleviate such a situation by tailoring, by means of the imprints made in the screen, the angular aperture of at least certain LEDs. It then becomes possible, for example, to decrease the number of LEDs of a type by increasing their emission power and by tailoring their angular aperture with the imprints made in the screen

The invention and its advantageous embodiments are defined in the appended set of claims. .

More specifically, the invention proposes a device for cosmetic treatment of the skin by light comprising a device for cosmetic treatment by light comprising:
- a plurality of light emission sources, each source emitting a light beam with a given angular aperture;
- a screen positioned facing the light sources and suitable for transmitting the light beams of each source,
in which the screen comprises at least one imprint integrated into the screen and modifying the angular aperture of the transmitted beam of at least one light emission source.

According to one embodiment, the plurality of light emission sources comprises at least one first group of sources emitting a light at a first wavelength and at least one second group of sources emitting a light at a second wavelength. For example, the number of light emission sources of the first group can be greater than the number of light emission sources of the second group. The light sources of the device can be surface-mounted on an electronic card.

According to one embodiment, the screen comprises an imprint for each light emission source. The screen can be opaque to the light beams outside of the imprints. The interior face of the screen can exhibit an anti-reflection treatment.

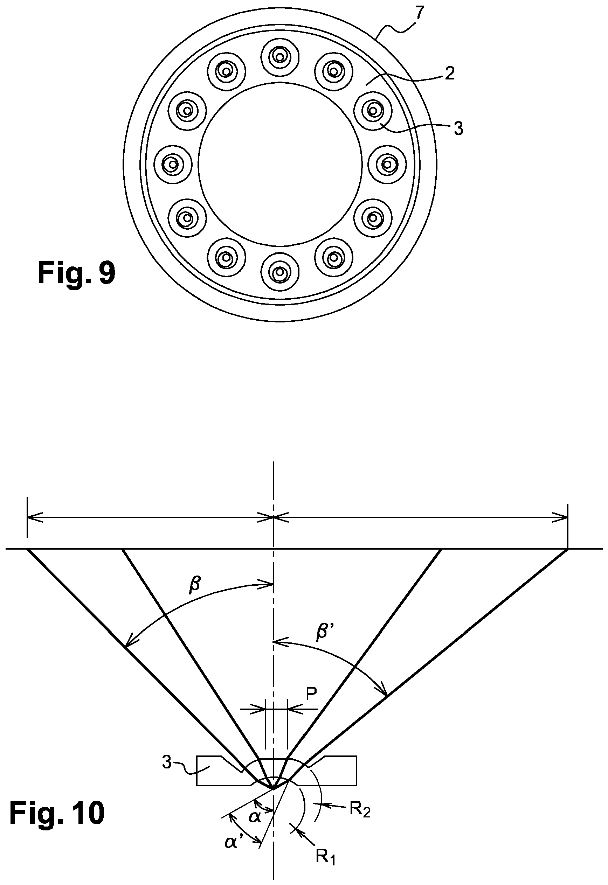

According to one embodiment, each imprint exhibits a first radius of curvature and at least one second radius of curvature, the first radius of curvature being defined so that the light beam emitted exhibits an angle of impact on the screen which is reduced. The term "angle of impact" is used herein in the sense of the angle of incidence, as is explained in Fig. 2 and Fig. 10. The invention also relates to an apparatus for cosmetic treatment, comprising a body and a device for cosmetic treatment by light according to the invention. Such an apparatus can exhibit various forms with a view to various applications. For example, the device for cosmetic treatment by light can be a nozzle suitable for being mounted in a reversible manner on the body of the apparatus; or the device can constitute an insert arranged on the body of the apparatus; or else the device can be housed in the body of the apparatus.

The subject of the invention is also an assembly - or kit - comprising a receptacle - pot, bottle, tube or other - of a cosmetic composition and an apparatus for cosmetic treatment by light according to the invention. The cosmetic composition of such a kit can be chosen from among a care product for oily skin and/or an anti-aging care product.

The subject of the invention is furthermore a cosmetic method comprising the implementation of the device for cosmetic treatment by light according to the invention. Advantageously, the cosmetic method comprises a step of applying a cosmetic composition, at least on an area of skin exposed to the luminous radiation of the device according to the invention.

### Brief description of the drawings

Other characteristics and advantages of the present invention will emerge on reading the detailed description which follows, given with reference to nonlimiting embodiments and illustrated by the appended drawings in which:
- Figure 1, already described, schematically illustrates the radiation distributions obtained with various types of light-emitting diodes;
- Figure 2 schematically illustrates a detailed partial view of a device for cosmetic treatment by light according to one embodiment of the invention;

- Figure 3 schematically illustrates a partial view of a device for cosmetic treatment by light according to the invention;
- Figure 4 schematically represents an area of skin treated by the device according to the invention;
- Figure 5 shows an exploded view of a first exemplary apparatus for cosmetic treatment according to the invention;
- Figure 6 shows the apparatus of Figure 5;
- Figure 7 shows a second exemplary apparatus for cosmetic treatment according to the invention;
- Figure 8 shows a third exemplary apparatus for cosmetic treatment according to the invention;
- Figure 9 shows an exemplary embodiment of a device for cosmetic treatment by light according to the invention; and
- Figure 10 schematically illustrates a detailed partial view of an imprint according to one embodiment of the invention.

The appended figures are provided solely by way of illustration and to facilitate the understanding of the invention. Not all the figures are represented to scale and certain elements of the devices and apparatuses described may not be represented in the figures.

### Detailed description of the invention

The invention relates to a device for cosmetic treatment by light. The device of the invention comprises a plurality of light emission sources (designated LEDs subsequently) and a screen positioned opposite the LEDs. The screen constitutes an interface of the device, that is to say an internal face of the screen is positioned facing the LEDs and an external face of the screen constitutes an exterior surface of the device. Each LED emits a light beam with a given angular aperture and this beam is transmitted through the screen to an area of skin to be treated. When several LEDs are used to cover an area of skin to be treated, the question of the homogeneity of the radiation over this area arises, especially at the periphery of the area.

Figure 2 shows a detail of a device according to one embodiment of the invention. Figure 1 depicts an LED 10 emitting a light beam 12 through a screen 2 toward an area of skin 30 to be treated. Figure 2 shows that the screen 2 exhibits an imprint 3 integrated into the actual thickness of the screen and which modifies the angular aperture of the beam 12 transmitted. The imprint 3 can be formed by laser etching for example or by molding during the fabrication of the screen. The LED 10 can be an HPLED. The LED can be monochromatic (i.e. its emission spectrum comprises a unique dominant wavelength peak), or multi-chromatic (i.e. its emission spectrum comprises several quasi-monochromatic lights, for example two).

The LED is designed to emit a light beam 12 with a given spectrum of wavelengths and a given angular aperture 2α. The LED can be designed to emit light in a continuous and/or pulsed manner in a given power range. The mode of emission and the emission power can be controlled by means of a microcontroller piloting the operation of the LED. On the other hand, the dominant wavelength or wavelengths and the angular aperture 2α of the beam emitted by the LED are generally specified by the constructor and not modifiable by the microcontroller.

The invention proposes to make provision for at least one imprint 3 in the screen 2 so as to modify the angular aperture 2α of the beam emitted by the LED and obtain an angular aperture 2β of the transmitted beam that is appropriate to the application envisaged.

According to one embodiment, the imprint 3 produced in the screen 2 exhibits a first radius of curvature R1 defining a concavity in the internal surface of the screen 2. This first radius of curvature R1 makes it possible to recover the peripheral rays emitted by the LED by deflection by making them converge. This first radius of curvature R1 is determined so as to obtain an angle of impact α' of the beam emitted of less than or equal to 45° whatever the angle of aperture 2α of the LED used. The imprint 3 thus makes it possible to limit the luminous reflections on the internal surface of the screen by reducing the angle of impact of the beam 12 on the screen 2. The imprint 3 produced in the screen 2 also exhibits a second radius of curvature R2 defining a convexity in the external surface of the screen 2. This second radius of curvature R2 is determined so that the modification of the angular aperture 2β of the beam 12 transmitted is that desired in the application of the device. For this purpose, the index n and the thickness m of the screen 2 at the level of the imprint 3 are known and controlled, as is the relative positioning of the screen 2 and of the focus of the LED 10 (designated by the distance e in Figure 2). One then knows precisely how the imprint 3 will modify the angular aperture of the beam 12 of the LED. The combination of the radii of curvature R1 and R2 makes it possible to render homogeneous the intensity of the light on the surface of the skin by modulation (convergent and/or divergent) of the rays emitted by the LED. The device according to the invention can also comprise a sensor of distance d from the area 30 to be treated so as to interrupt the emission of the LEDs when the distance d becomes too small - through safety - or too large - through lack of effectiveness.

Provision may thus be made for one or more imprints 3 in the screen 2. It is possible to provide for an imprint per LED or an imprint for certain LEDs of the device only, for example those situated at the periphery or those emitting at a certain wavelength. In Figure 2, the imprint 3 has tightened the light beam 12 (the angular aperture 2β of the beam transmitted is less than the initial angular aperture 2α of the beam emitted), but it is possible to envisage an inverse radius of curvature R2 which would widen the beam. Likewise, it would be possible to provide for an imprint 3 which exhibits only a single radius of curvature R1 or R2. For example, the internal surface of the screen could remain plane if the sources 10 used exhibit an insignificant initial angular aperture, or else the external surface of the screen could remain plane if the convergence afforded by the radius of curvature of the internal surface is sufficient.

In one embodiment, the device for cosmetic treatment by light according to the invention can comprise at least two groups of LEDs emitting respectively at two different wavelengths. When two types of LED emitting at two different wavelengths are used in a concomitant manner, it is necessary to guarantee a precise power of luminous radiation per unit of exposed skin for each type of wavelength.

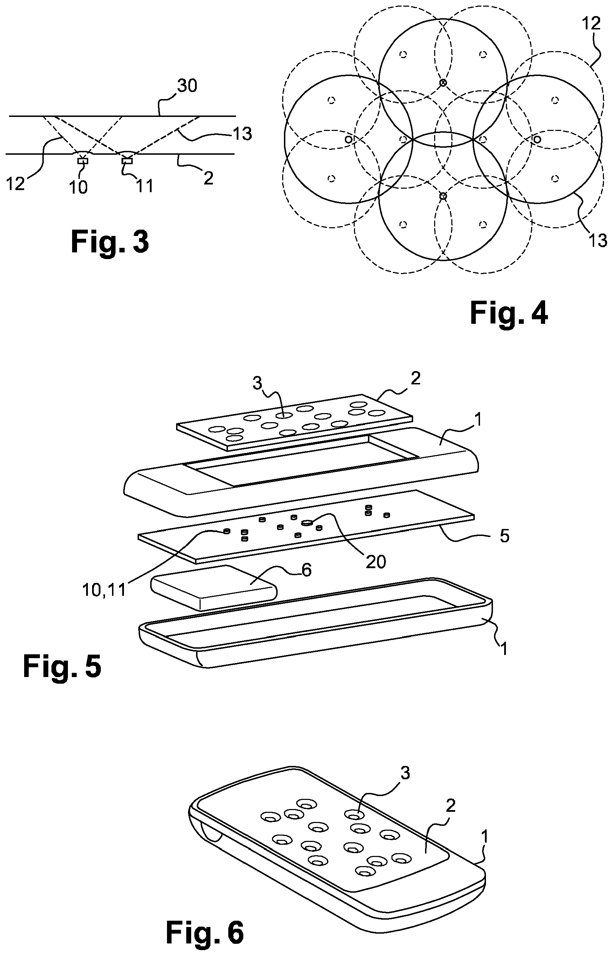

Figure 3 shows an exemplary application of the device according to the invention when two types of LED are used in a concomitant manner. Figure 2 shows two LEDs 10, 11 of two distinct types each emitting a light beam 12, 13 through the screen 2 toward the area of skin 30 to be treated. Figure 3 shows that the screen 2 exhibits an imprint 3 for each LED which modifies in a distinct manner the angular aperture of each beam 12, 13 transmitted. More specifically, each imprint 3 produced in the screen 2 exhibits radii of curvature determined so that the modification of the angular aperture of each beam 12, 13 transmitted is that desired in the application of the device.

For example, the radii of curvature of the imprints 3 can be determined so that the angular aperture of the transmitted beams 12 of a first type of LED 10 is smaller than the angular aperture of the transmitted beams 13 of a second type of LED 11. It then becomes possible to ensure the homogeneity of the radiations of the two types of LED over the area to be treated without necessarily using the same number of LEDs of each type.

Figure 4 illustrates an example in which the LEDs of a first type are more than doubled with respect to the LEDs of a second type even though the area to be treated remains substantially covered in the same manner by the radiations of the two types of LED. It is thus possible to optimize the costs while not using the same number of LEDs of each type. For example, it is possible to envisage exposing an area of skin to a first electroluminescent radiation by means of ten LEDs emitting light beams 12 at the wavelength of 590 nm with a power of 2.1 mW/cm² of exposed skin, and simultaneously, expose the area of skin to a second electroluminescent radiation by means of four LEDs emitting light beams 13 at the wavelength of 870 nm with a power of 0.5 mW/cm² of exposed skin.

For example, HPLEDs can be used as first type of light sources and conventional LEDs can be used as second type of light sources. The screen 2 can exhibit imprints 3 just for the LEDs of the first type of light sources, or imprints 3 for the LEDs of each type of light source, with radii of curvature appropriate to the type of LED and/or to the location of each LED in the device.

Figures 5 and 6 illustrate an exemplary application in which the device for cosmetic treatment by light is integrated into an apparatus; Figure 5 is an exploded view and Figure 6 is a view of the apparatus assembled.

Figure 5 shows that the LEDs 10, 11 can be arranged on an electronic card 5, for example by using LEDs of "surface mounted components" (SMC) type. The electronic card 5 can also carry a distance sensor 20 suitable for measuring the distance d mentioned with reference to Figure 1. The apparatus exhibits a body 1 consisting of a shell exhibiting a front face furnished with the screen 2 and a rear face. The shell of the body 1 makes it possible to house the card 5 carrying the LEDs 10, 11 as well as other electronic components and a battery 6. The electronic card 5 can in particular carry a microcontroller which controls the modes of operation of the apparatus according to programs activated by the user by means of an interface provided for on the body 1. Any type of program can be envisaged within the framework of this invention to control pulsed and/or continuous and/or alternated emissions of light. The microcontroller can also control the operation of the apparatus as a function of the data received from the distance sensor 20, for example control the stopping of the light emission of the LEDs even though the program is in progress if the distance between the screen 2 and the area to be treated becomes too large or too small.

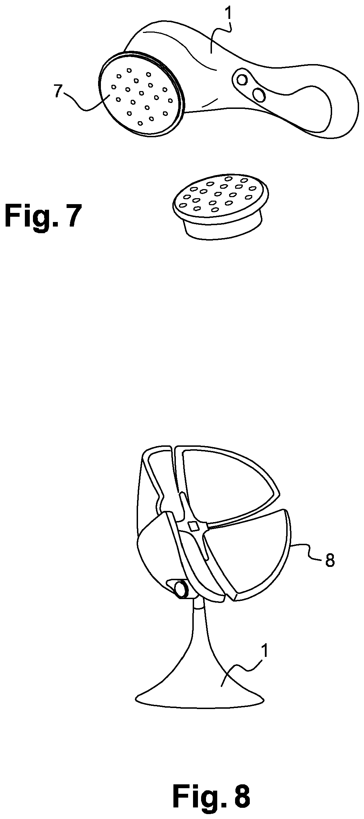

The imprints 3 in the screen 2 can be seen in Figures 5 and 6. The imprints 3 can exhibit an external diameter of between 1.5 mm and 6 mm. According to one embodiment, the screen 2 can be rendered opaque, by sand-blasting for example, outside of the imprints 3. The light beams of the LEDs can still pass through the screen but the electronic card 5 and the various components remain hidden from the user's eyes. The imprints 3 may optionally be tinted to constitute patches of colors on the screen 2. The distribution of the imprints 3 depends on the applications. The imprints can be arranged in groups as in Figure 6 or distributed evenly over the whole of the surface of the screen 2 as in Figure 7. The internal surface of the screen 2 can be coated with an anti-reflection treatment to attenuate any reflection of the beams of the LEDs inside the device.

Figure 7 illustrates another exemplary application in which the device for cosmetic treatment by light constitutes a nozzle 7 that can be fitted and removed. The apparatus comprises a body 1 which can exhibit the form of a handle and which can house a battery and a microcontroller for controlling the operation of the LEDs. According to an alternative, the microcontroller could be situated on an electronic card housed in the nozzle 7.

Figure 8 illustrates yet another exemplary application in which the device for cosmetic treatment by light constitutes an insert 8 arranged on the apparatus. The apparatus comprises a body 1 which can exhibit the form of a support and which can house a battery and a microcontroller for controlling the operation of the LEDs. In Figure 8, it is seen that a plurality of devices 8 is inserted on the support 1.

### Example 1

According to a first exemplary implementation, ten LEDs at 590 nm are used, exhibiting an initial angular aperture (designated 2α in Figure 2) of 120° and four LEDs at 870 nm are used, exhibiting an initial angular aperture 2α of 140°. The distance (designated e in Figure 2) between the screen and the LEDs is 0.5 mm and the distance (designated d in Figure 2) between the LEDs and the area of skin to be treated is recommended at 20 mm. An imprint is provided for each LED. The internal radius (designated R1 in Figure 2) of each imprint is 3.5 mm and the external radius (designated R2 in Figure 2) of each imprint is 5.5 mm. The screen exhibits a refractive index of 1.586 and the thickness (designated m in Figure 2) of each imprint is 1.8 mm for the imprints associated with the LEDs at 590 nm and 1.9 mm for the imprints associated with the LEDs at 870 nm. The more significant thickness of the imprints associated with the LEDs at 870 nm makes it possible to reduce the convergence. Such an arrangement makes it possible to obtain, for each of the ten LEDs at 590 nm, an angle of impact (designated α' in Figure 2) of 41° and an angular aperture of the beam transmitted (designated 2β in Figure 2) of 108°; and for each of the four LEDs at 870 nm, an angle of impact α' of 45° and an angular aperture of the beam transmitted 2β of 128°. Such an arrangement makes it possible to treat in a homogeneous manner with two different types of radiation an area of skin of about 50 cm².

### Example 2

According to a second exemplary implementation, the LEDs can be arranged as a ring as illustrated in Figure 9, for example in a nozzle 7 intended for an apparatus of the type described with reference to Figure 7. Six LEDs at 590 nm are used, exhibiting an initial angular aperture 2α of 120° and three LEDs at 870 nm are used, exhibiting an initial angular aperture 2α of 140°. The distance between the screen and the LEDs is 0.5 mm and the distance between the LEDs and the area of skin to be treated is recommended at 20 mm. An imprint is provided for each LED; and for esthetic reasons, the number of imprints can be greater than the number of LEDs so as to exhibit an even ring-like distribution on the surface of the nozzle 7.

On account of the ring-like arrangement of the LEDs, to obtain good homogeneity of the luminous intensity over the exposed area of skin, it is sought to increase the intensity of the light in the external part of the ring and to reduce substantially the intensity of the light in the internal part of the ring. The shape of the imprint 3 is then adapted as illustrated in Figure 10. Each imprint 3 exhibits an internal radius R1 of 3.5 mm; and the external surface of each imprint 3 combines various radii and a plane surface so as to increase the intensity of the light on the external area. The external radius R2 is 2.5 mm with a plane area P of diameter 1.7 mm and off-centered with respect to the axis of emission of the LED toward the interior of the ring. The screen exhibits a refractive index of 1.586 and the thickness of each imprint is 1.5 mm for the imprints associated with the LEDs at 590 nm and 1.6 mm for the imprints associated with the LEDs at 870 nm. The more significant thickness of the imprints associated with the LEDs at 870 nm makes it possible to reduce the convergence.

Such an arrangement makes it possible to obtain, for each of the six LEDs at 590 nm, an angle of impact α' of 38° and an angular aperture of the beam transmitted of β=44.5° toward the exterior of the ring and of β'=50.8° toward the interior of the ring; and for each of the three LEDs at 870 nm, an angle of impact α' of 42° and an angular aperture of the beam transmitted of β=49° toward the exterior of the ring and of β'=55.6° toward the interior of the ring. Such an arrangement makes it possible to treat in a homogeneous manner with two different types of wavelength an area of skin of about 32 cm².

The present invention has been described with reference to particular embodiments illustrated in Figures 1 to 10, but it is understood that other variants can be envisaged by the person skilled in the art, especially the number of types of LED having different wavelengths can be greater than two and arrangements and dimensions other than those described in Figures 3 to 10 can be envisaged to constitute devices according to the invention.

## Claims

1. A device for a cosmetic skin treatment by light, comprising:
- a plurality of light emission sources (10, 11), each source emitting a light beam (12, 13) with an angular aperture (2α);
- a screen (2) positioned opposite and facing the light sources (10, 11) and suitable for transmitting the light beams of each source through the screen to an area of the skin to be treated,
in which the screen (2) comprises at least one imprint (3) integrated into the actual thickness of screen and acting as lenses so as to modify the angular aperture (2β) of the transmitted beam of at least one light emission source,
each imprint (3) exhibiting a first radius of curvature (R1) defining a concavity in an internal surface of the screen facing the at least one light source, and at least one second radius of curvature (R2) defining a convexity in the external surface of the screen (2), the first radius of curvature (R1) being defined so that the light beam (12, 13) emitted exhibits an angle of impact (α') on the screen which is less than or equal to 45°.

2. The device as claimed in claim 1, in which the plurality of light emission sources (10, 11) comprises at least one first group of sources (10) emitting a light at a first wavelength (λ₁) and at least one second group of sources (11) emitting a light at a second wavelength (λ₂).

3. The device as claimed in claim 2, in which the number of light emission sources (10) of the first group is greater than the number of light emission sources (11) of the second group.

4. The device as claimed in any one of the preceding claims, in which the screen (2) comprises an imprint (3) for each light emission source (10, 11).

5. The device as claimed in claim 4, in which the screen (2) is opaque to the light beams (12, 13) outside of the imprints (3).

6. The device as claimed in any one of the preceding claims, in which the interior face of the screen (2) exhibits an anti-reflection treatment.

7. The device as claimed in any one of the preceding claims, in which the light sources (10, 11) of the device are surface-mounted on an electronic card (5).

8. An apparatus for cosmetic treatment, comprising a body (1) and a cosmetic treatment device as claimed in any one of claims 1 to 7.

9. The apparatus as claimed in claim 8, in which the device is housed in the body (1) of the apparatus.

10. The apparatus as claimed in claim 8, in which the device constitutes a nozzle (7) suitable for being mounted in a reversible manner on the body (1).

11. The apparatus as claimed in claim 8, in which the device constitutes an insert (8) arranged in a support of the body (1).

12. An assembly, comprising:
- a receptacle of a cosmetic composition;
- a cosmetic treatment apparatus as claimed in any one of claims 8 to 11.

13. A non-therapeutic cosmetic method, comprising the implementation of the device as claimed in any one of claims 1 to 7.

14. The cosmetic method as claimed in claim 13, comprises a step of applying a cosmetic composition, at least on an area of skin exposed to the luminous radiation of the device.

## Patentansprüche

1. Vorrichtung für eine kosmetische Hautbehandlung mit Licht, umfassend:
- eine Vielzahl von Lichtemissionsquellen (10, 11), wobei jede Quelle einen Lichtstrahl (12, 13) mit einer Winkelapertur (2α) emittiert;
- einen Schirm (2), der den Lichtquellen (10, 11) gegenüberliegend und zugewandt angeordnet ist und geeignet ist, die Lichtstrahlen jeder Quelle durch den Schirm auf einen zu behandelnden Bereich der Haut durchzulassen,
wobei der Schirm (2) wenigstens eine Vertiefung (3) umfasst, die in die effektive Dicke des Schirms aufgenommen ist und als Linse wirkt, um die Winkelapertur (2β) des durchgelassenen Strahls wenigstens einer Lichtemissionsquelle zu modifizieren,
jede Vertiefung (3) einen ersten Krümmungsradius (R1) aufweist, der eine Konkavität in einer inneren Oberfläche des Schirms, die der wenigstens einen Lichtquelle zugewandt ist, definiert, und wenigstens einen zweiten Krümmungsradius (R2), der eine Konvexität in der äußeren Oberfläche des Schirms (2) definiert, wobei der erste Krümmungsradius (R1) so definiert ist, dass der emittierte Lichtstrahl (12, 13) einen Einfallswinkel (α') auf den Schirm aufweist, der kleiner als oder gleich 45° ist.

2. Vorrichtung gemäß Anspruch 1, wobei die Vielzahl von Lichtemissionsquellen (10, 11) wenigstens eine erste Gruppe von Quellen (10), die Licht mit einer ersten Wellenlänge (λ₁) emittieren, und wenigstens eine zweite Gruppe von Quellen (11), die Licht mit einer zweiten Wellenlänge (λ₂) emittieren, umfasst.

3. Vorrichtung gemäß Anspruch 2, wobei die Anzahl von Lichtemissionsquellen (10) der ersten Gruppe größer als die Anzahl von Lichtemissionsquellen (11) der zweiten Gruppe ist.

4. Vorrichtung gemäß einem der vorstehenden Ansprüche, wobei der Schirm (2) eine Vertiefung (3) für jede Lichtemissionsquelle (10, 11) aufweist.

5. Vorrichtung gemäß Anspruch 4, wobei der Schirm (2) außerhalb der Vertiefungen (3) für die Lichtstrahlen (12, 13) undurchlässig ist.

6. Vorrichtung gemäß einem der vorstehenden Ansprüche, wobei die Innenseite des Schirms (2) eine Antireflexbehandlung aufweist.

7. Vorrichtung gemäß einem der vorstehenden Ansprüche, wobei die Lichtquellen (10, 11) der Vorrichtung auf einer Elektronikkarte (5) oberflächenmontiert sind.

8. Gerät für kosmetische Behandlung, umfassend einen Körper (1) und eine kosmetische Behandlungsvorrichtung gemäß einem der Ansprüche 1 bis 7.

9. Gerät gemäß Anspruch 8, wobei die Vorrichtung in dem Körper (1) des Geräts untergebracht ist.

10. Gerät gemäß Anspruch 8, wobei die Vorrichtung eine Düse (7) bildet, die geeignet ist, auf reversible Weise an dem Körper (1) angebracht zu werden.

11. Gerät gemäß Anspruch 8, wobei die Vorrichtung einen Einsatz (8) bildet, der dafür gestaltet ist, in einem Träger des Körpers (1) angeordnet zu werden.

12. Baugruppe, umfassend:
- einen Behälter für eine kosmetische Zusammensetzung;
- ein kosmetisches Behandlungsgerät gemäß einem der Ansprüche 8 bis 11.

13. Nichttherapeutisches kosmetisches Verfahren, umfassend die Anwendung der Vorrichtung gemäß einem der Ansprüche 1 bis 7.

14. Kosmetisches Verfahren gemäß Anspruch 13, umfassend einen Schritt des Aufbringens einer kosmetischen Zusammensetzung wenigstens auf einen Bereich von gegenüber der Lichtstrahlung der Vorrichtung exponierter Haut.

## Revendications

1. Dispositif de traitement cosmétique par la lumière, comprenant :
- une pluralité de sources d'émission de lumière (10, 11), chaque source émettant un faisceau lumineux (12, 13) ayant une ouverture angulaire (2α) ;
- un écran (2) positionné en regard et en face des sources de lumière (10, 11) et adapté à transmettre les faisceaux lumineux de chaque source à travers l'écran vers une zone de la peau à traiter,
dans lequel l'écran (2) comprend au moins une empreinte (3) intégrée dans l'épaisseur même de l'écran et faisant office de lentilles pour modifier l'ouverture angulaire (2β) du faisceau transmis d'au moins une source d'émission de lumière,
chaque empreinte (3) présentant un premier rayon de courbure (R1) définissant une concavité dans une surface interne de l'écran en face de la au moins une source de lumière, et au moins un deuxième rayon de courbure (R2) définissant une convexité dans la surface externe de l'écran (2), le premier rayon de courbure (R1) étant défini pour que le faisceau lumineux (12, 13) émis présente un angle d'impact (α') sur l'écran inférieur au égal à 45°.

2. Dispositif selon la revendication 1, dans lequel la pluralité de sources d'émission de lumière (10, 11) comprend au moins un premier groupe de sources (10) émettant une lumière à une première longueur d'onde (λ₁) et au moins un deuxième groupe de sources (11) émettant une lumière à une deuxième longueur d'onde (λ₂).

3. Dispositif selon la revendication 2, dans lequel le nombre de sources d'émission de lumière (10) du premier groupe est supérieur au nombre de sources d'émission de lumière (11) du deuxième groupe.

4. Dispositif selon l'une quelconque des revendications précédentes, dans lequel l'écran (2) comprend une empreinte (3) pour chaque source d'émission de lumière (10, 11).

5. Dispositif selon la revendication 4, dans lequel l'écran (2) est opaque aux faisceaux lumineux (12, 13) en dehors des empreintes (3).

6. Dispositif selon l'une quelconque des revendications précédentes, dans lequel la face intérieure de l'écran (2) présente un traitement antireflet.

7. Dispositif selon l'une quelconque des revendications précédentes, dans lequel les sources de lumière (10, 11) du dispositif sont montées en surface sur une carte électronique (5).

8. Appareil pour traitement cosmétique, comprenant un corps (1) et un dispositif de traitement cosmétique selon l'une quelconque des revendications 1 à 7.

9. Appareil selon la revendication 8, dans lequel le dispositif est logé dans le corps (1) de l'appareil.

10. Appareil selon la revendication 8, dans lequel le dispositif constitue un embout (7) adapté à être monté de manière réversible sur le corps (1).

11. Appareil selon la revendication 8, dans lequel le dispositif constitue un insert (8) agencé dans un support du corps (1).

12. Ensemble comprenant :
- un réceptacle d'une composition cosmétique ;
- un appareil de traitement cosmétique selon l'une quelconque des revendications 8 à 11.

13. Procédé cosmétique non thérapeutique comprenant la mise en oeuvre du dispositif selon l'une quelconque des revendications 1 à 7.

14. Procédé cosmétique selon la revendication 13, comprenant une étape d'application d'une composition cosmétique, au moins sur une zone de peau exposée au rayonnement lumineux du dispositif.
